# EUROPEAN PATENT APPLICATION

(11) **EP 2 722 067 A1**
(43) Date of publication of application: **23.04.2014**
(21) Application number: 12188841.6
(22) Date of filing: 17.10.2012
(51) Int. Cl.: A61M 5/24

(54) **Single-use drug reservoir connector**

(71) Applicant: Sensile Pat AG, 4614 Haegendorf (CH)
(72) Inventor: Marbet, Regina, 4933 Rütschelen (CH); Kladiwa, Malte, CH-3008 Bern (CH)
(74) Representative: reuteler & cie SA

(57) **Abstract**

A single-use drug reservoir connector for use in a medical device, including a drug transfer system (4) comprising a cannula (12), a docking system (6) and a blocking mechanism (20) comprising a blocking portion (46). The docking system is configured to receive and hold a reservoir (2) and comprises a reservoir holding mechanism (16) and a receiving interface element (18). The receiving interface element comprises a complementary blocking portion (48), the receiving interface element being configured to be moved from a first position, in which the cannula (12) is fluidically connected to the reservoir, to a second position, in which the cannula is disconnected from the reservoir. The receiving interface element engages the blocking portion in the second position via the complementary blocking portion, the blocking portion is configured to prevent the receiving interface element from being moved from the second position to the first position.

## Description

The present invention relates to a single use drug reservoir connector for use in medical devices. The invention may in particular be used in medical devices comprising multiple reservoirs containing different drugs or different drug components.

Various injection systems and devices use pre-filled reservoirs comprising a drug such as for example insulin or the like. The reservoirs are configured to be fluidically connected with a needle cannula to transfer the drug and comprise in general a plunger on one side and a cap with a septum on the other side. When the drug in the reservoir has to be injected, the needle cannula pierces the septum and thus enables the transfer of the liquid drug. The cannula and all the parts coming in direct contact with the drug or body fluid of a patient are single-use. Especially the needle cannula, which comes in direct contact with body fluid during injection, should be disposed after the each injection operation. Other parts of the injection system or device, which came in contact with the drug, may be used more than once but should be replaced when the reservoir comprising the drug is empty and a new reservoir is put in place.

Some existing concepts try to avoid the re-use of various parts by designing devices that for example do not allow the disconnection of the reservoir without destroying the medical device. This can be achieved for instance by an irreversible snapping mechanism arranged on an adapter, the snapping mechanism being configured to irreversibly lock the reservoir to the adapter, once it is inserted. The adapter can for example comprise the needle cannula used for piercing of the septum and for injection of the drug. When the reservoir is empty, the medical device or at least the adapter together with the reservoir has to be disposed.

Another solution of avoiding the re-use of parts of medical devices that come in contact with the drug or body fluid is the use of fully disposable pens comprising a pre-filled reservoir or cartridge. Such fully-disposable pens cannot be opened without being destroyed and thus they do not allow the exchange of the reservoir or cartridge. After the injection of the drug the fully-disposable pen is disposed as a whole.

In some cases the above described devices have to be thrown away before the reservoir or cartridge comprising the drug is actually empty, wasting the drug that is still in the reservoir. This might be the case especially when the medical device comprises two or more reservoirs with different drugs or drug components that have to be mixed prior to injection. Since in some cases these drug components have to be mixed in an exact ratio it is likely that one reservoir used with the medical device is empty prior to another reservoir used with the same medical device. If the medical device, such as for example a dual drug pen, does not allow the removal of the reservoir still comprising drug or drug component, the drug or drug component will go to waste. As some drugs are very expensive it is not desirable to waste a reservoir still comprising a drug or drug component.

In a drug reconstitution device configured to dissolve a lyophilized drug with a liquid, for instance as described in patent application CH 00337/12 incorporated herein by reference, it is desirable to provide a connector for releasably connecting the containers or reservoirs to the disposable liquid transfer unit. If a container is releasably connected to the liquid transfer unit it is however of advantage to ensure that the liquid transfer unit cannot be used more than once to reconstitute a drug.

An object of this invention is to provide a single-use drug reservoir connector, which is economical to use, safe, reliable and allow the disconnection of a reservoir.

It is advantageous to provide a single-use drug reservoir connector that is versatile and compact.

It is advantageous to provide a single-use drug reservoir connector that is robust and easy to use.

It is advantageous to provide a single-use drug reservoir connector that is flexible and can be used in various medical applications.

Disclosed herein is a single-use drug reservoir connector including a drug transfer system comprising a cannula, a docking system and a blocking mechanism comprising a blocking portion. The docking system is configured to receive and hold a reservoir, the docking system comprising a reservoir holding mechanism and a receiving interface element comprising a complementary blocking portion. The receiving interface element is configured to be moved from a first position, in which the cannula is fluidically connected to the reservoir, to a second position, in which the cannula is fluidically disconnected from the reservoir. The receiving interface element engages the blocking portion in the second position via the complementary blocking portion. The blocking portion is configured to prevent the receiving interface element from being moved from the second position back to the first position.

Since the receiving interface element cannot be moved from the second position to the first position, the re-use of the cannula, which was in contact with the drug and most likely with the body fluid, is not possible.

In an advantageous embodiment the reservoir holding mechanism comprises at least one latching element configured to releasably hold the reservoir.

The latching element is elastic so that the reservoir can be taken away from the reservoir holding mechanism by overcoming a retention force but without excessive force, by a patient or a user. This allows the re-use of the reservoir in case there is still drug or drug component contained in the reservoir.

In an advantageous embodiment the latching element is integrally formed with the receiving interface element.

It is possible to fixedly connect the latching element to the receiving interface element. Since the reservoir is configured to be connected to the reservoir holding mechanism and the latching arm respectively, when the receiving interface element is in the first position so that the cannula may pierce the reservoir, the latching element ensures via the fixed connection to the receiving interface element that it is moved from the first position to the second position by a user when the reservoir is removed. The force needed to move the receiving interface element from the first position to the second position is smaller than the retention force of the reservoir holding mechanism.

In an advantageous embodiment the cannula protrudes from the receiving interface element when it is in the first position and it is covered by the receiving interface element when it is in the second position.

The cannula may extend through a recess of the receiving interface element and protrude from it to pierce a reservoir, when the receiving interface element is in the first position. The receiving interface element is moved into the second position, preferably by pulling of the reservoir away from the cannula by a user. When the receiving interface element is in the second position the cannula is shielded by the receiving interface element and cannot pierce the reservoir any longer.

The distance from the first position to the second position, measured along the longitudinal direction of the cannula, is longer than the protruding part of the cannula.

The single-use drug reservoir connector may further comprise a body portion, in which the reservoir holding mechanism, the receiving interface element and parts of the drug transfer system is arranged.

In an advantageous embodiment, the body portion may comprise a stop member configured to prevent the receiving interface portion from moving beyond the second position.

The stop member is configured to engage the receiving interface element, when it moves beyond the second position, thus limiting a movement in a direction of removal of the reservoir. When a user wants to remove the reservoir the stop member ensures that a force can be applied to the reservoir holding mechanism so that the retention force may be overcome and thus the reservoir is released by the reservoir holding mechanism.

In another advantageous embodiment the blocking mechanism comprises an elastic blocking element comprising the blocking portion.

The elastic blocking element is configured to engage the complementary blocking portion of the receiving interface element once the complementary blocking portion is moved past the blocking portion, when the receiving interface element is moved from the first to the second position.

The elastic blocking element may comprise an elastic cantilever arm or a mechanism comprising a slide bolt and a spring that engages and moves the slide bolt as soon as the receiving interface element is moved past the bolt. The spring and the blocking element may have various other configurations within the scope of the invention that accomplish the function of an elastically movable blocking portion allowing displacement of the receiving interface element past the blocking portion in one direction, but blocks it in the other direction.

In an advantageous embodiment the elastic blocking element may comprise a camming surface configured to move the elastic blocking element or at least parts of it aside, when the receiving interface element is moved from the first to the second position. The camming surface is further configured to release the elastic blocking element latest when the receiving interface element is in the second position.

Further objects and advantageous features of the invention will be apparent from the claims, from the detailed description, and annexed drawings, in which:
Fig. 1 is a cross sectional view of an embodiment of a single-use drug reservoir connector according to the invention with a receiving interface element in a first position;
Fig. 2 is a bottom-up view on a cross section cut along line II-II of the embodiment according to figure 1;
Fig. 3 is a bottom-up view on a cross section cut along line III-III of the embodiment according to figure 1;
Fig. 4 is another cross sectional view of an embodiment according to the invention;
Fig. 5 a perspective view of an embodiment according to the invention;
Fig. 6 is another perspective view of an embodiment according to the invention;
Fig. 7 is cross sectional view of an embodiment according to figure 1 with a receiving interface element being arranged in a second position;
Fig. 8 is a cross sectional view of an embodiment according to the invention comprising a reservoir;
Fig. 9 is another cross sectional view of an embodiment according to the invention comprising the reservoir;
Fig. 10 is a cross sectional view of an embodiment according to the invention with the receiving interface element in the second position; and
Fig. 11 is another cross sectional view of an embodiment according to the invention comprising a reservoir that has been emptied.

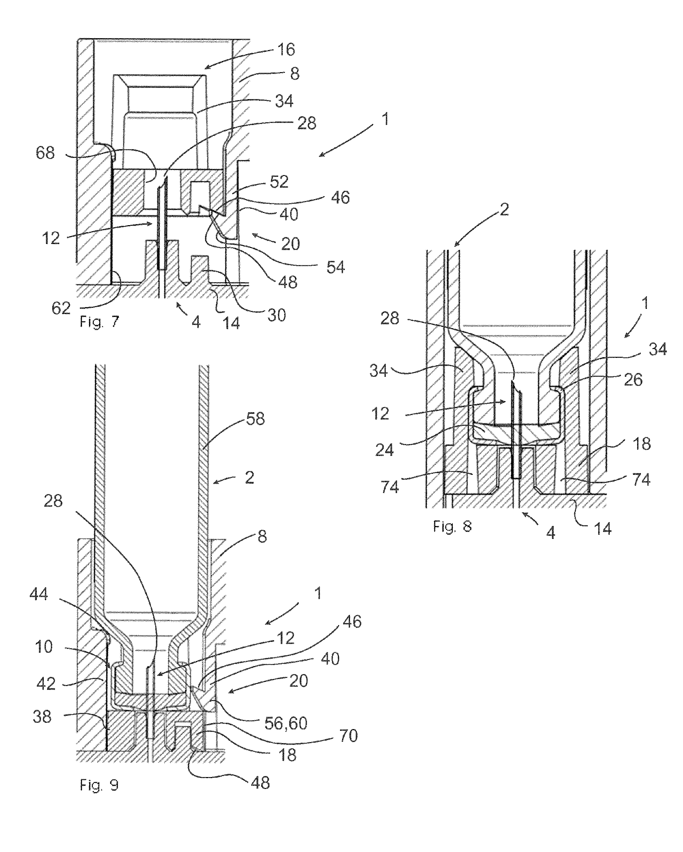

Referring to the figures, which illustrate an embodiment of a single-use drug reservoir connector 1, the single-use drug reservoir connector comprises a body portion 8 in which a docking system 6 and a blocking mechanism 20 are arranged. A part of a drug transfer system 4 comprising a cannula 12 is introduced into a cavity 62 of the body portion 8. The drug transfer system 4 is not entirely embedded in the cavity 62 but at least partially so that the cannula 12 may extend into the cavity 62 and pierce a reservoir 2 that can be connected to the docking system 6, as shown in figures 8 to 11. The drug transfer system 4 comprises a support 14 with a positioning member 30 and base 32.

The support 14 is attached to the body portion 8, either fixedly or releasably. The support 14 may even be integrally formed with the body portion 8. However in certain applications it might be desirable to be able to disconnect the body portion 8 from the drug transfer system 4, for example when the cannula 12 is part of the body portion 8 and thus is configured to be removed and disposed together with the body portion 8 and the parts arranged therein.

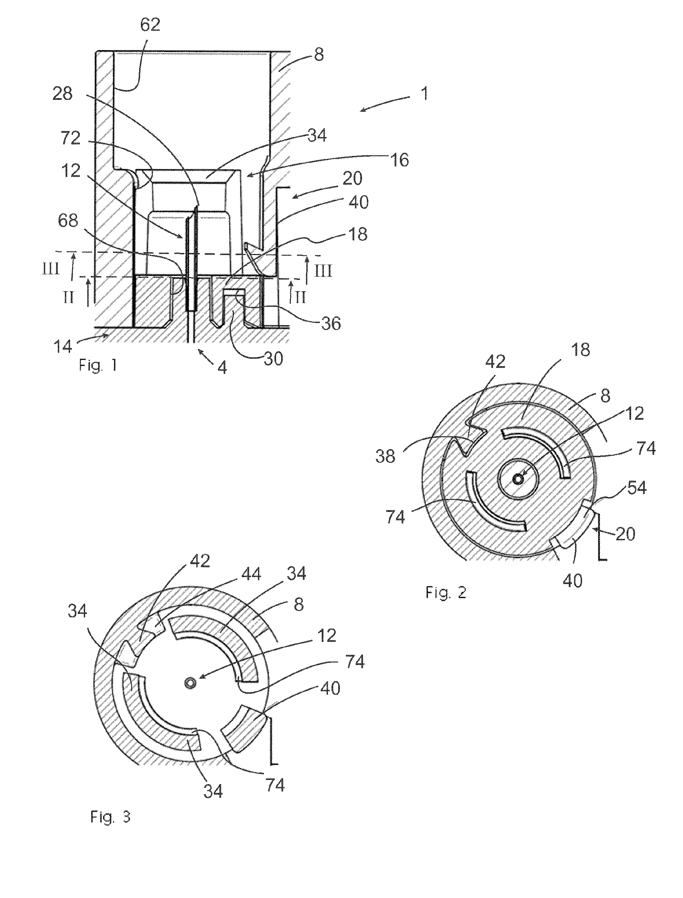

The docking system 6 comprises a reservoir holding mechanism 16 and a receiving interface element 18. The reservoir 2 is configured to be installed or clipped into the reservoir holding mechanism 16 when the receiving interface element 18 is in a first position. The receiving interface element 18 is illustrated in a first position in figures 1 to 6, 8 and 9.

Referring to the figures, in an embodiment the receiving interface element 18 is generally shaped as a disc, for instance as a circular disc, comprising a reservoir supporting interface 64 oriented towards an open end 66 of the cavity 62. The reservoir supporting interface 64 is configured to support a cap 10 of a reservoir 2, when the reservoir 2 is installed in the docking system 6, as shown in figures 8 to 11. The receiving interface element 18 comprises a passage 68 allowing the cannula 12 and the base 32, in which the cannula 12 is embedded, to pass through, so that the cannula 12 comprising a piercing tip 28 at its free end is protruding from the reservoir supporting interface 64 when the receiving interface element 18 is in the first position as illustrated in figures 1 to 6, 8 and 9. The piercing tip 28 is configured to pierce a septum 24 of the reservoir 2 comprising the drug or drug component when the reservoir 2 is installed in the docking system 6.

In the embodiment illustrated the receiving interface element 18 is shaped as a circular plate; however other shapes such as for example a square plate fall within the scope of the invention.

The support 14 further comprises a positioning member 30, which is in the shape of a protrusion, configured to engage a complementary positioning member 36 of the receiving interface element 18 to ensure a fix and stable positioning of the receiving interface element 18 within the cavity 62 of the body portion 8. The complementary positioning member 36 is in the shape of a recess. The positioning member 30 and the complementary positioning member 36 are used during production, to hold the position of the receiving interface element 18 relative to the support 14. Other shapes of positioning member 30 and complementary positioning member 36 fall within the scope of the invention.

Referring now to figures 7 and 10 to 11, where the receiving interface element 18 is in the second position, the piercing tip 28 of the cannula 12 does not protrude any longer from the receiving interface element 18 and the reservoir receiving surface 64, respectively. When the receiving interface element 18 is in the second position, the piercing tip 28 can no longer pierce a septum 24 of a reservoir 2. The receiving interface element 18 is shielding the piercing tip 28.

Initially, before connection of the reservoir 2 to the single-use drug reservoir connector 1 the receiving interface element 18 is in the first position, at least partially resting on the support 14 of the drug transfer system 4. This is however not necessary, since there might be an extra stop element that acts as a base for the receiving interface element 18 or other means that limit the movement of the receiving interface element 18 towards the support 14. In the first position, the reservoir 2 may be easily connected to the single-use drug reservoir connector 1. The reservoir 2 may be introduced, for example by a patient or medical personnel, and connected to the single-use drug connector 1 through an open end of the cavity 62 onto the cannula 12 which will pierce the septum 24 with the piercing tip 24 and onto the reservoir supporting interface 64 of the receiving interface element 18. The holding mechanism 16 comprises latching arms 34, in the figures an embodiment comprising two latching arms 34 is shown, which may engage, for example via a shoulder as illustrated in figure 8, a collar or a flange 26 of the cap 10 of the reservoir 2 when the reservoir 2 is connected to the single-use drug reservoir connector 1. Upper edges of the latching arms 34 may be configured to support a shoulder portion of the reservoir 2 to provide more stability to the connection between docking mechanism 6 and reservoir 2. The latching arms 34 may be best seen in figures 1, 3 and 4 to 8. The latching arms 34 are elastic and configured to being bent out when the cap 10 of the reservoir 2 is slid past them and snap in towards the collar 26 of the cap 10 when the reservoir 2 is fully engaged in the docking mechanism 6. Since the latching arms 34 of the reservoir holding mechanism 6 are elastic a retention force may be established between the reservoir 2 and the latching arms 34. The retention force may not be too strong, since it has to be possible to overcome it by a user in order to manually remove the reservoir 2.

Once the reservoir 2 is engaged and connected to the single-use drug reservoir connector 1, the administration, mixing, reconstitution or pumping of the drug may start.

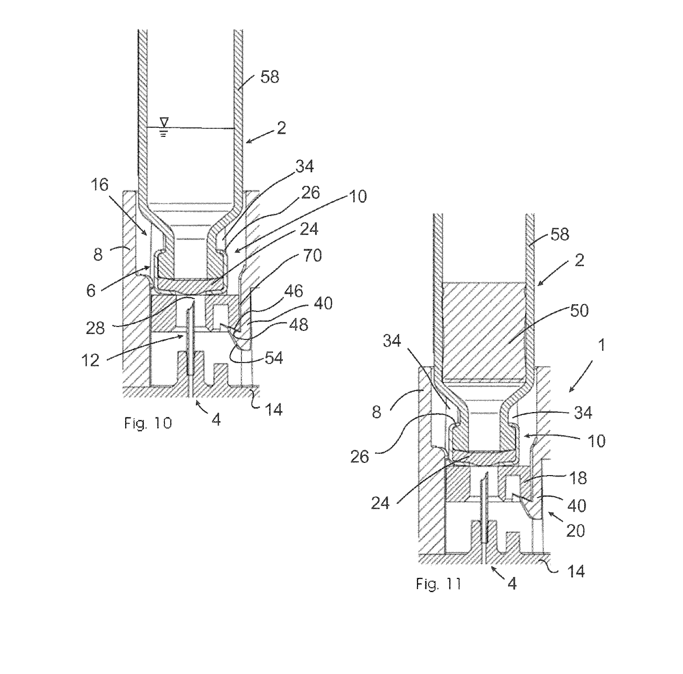

When the administration, mixing, or treatment of the drug or drug component is done the patient or medical personnel may move the receiving interface element 18 from the first position to the second position by pulling the reservoir 2 away from the cannula 12, as shown in figures 10 and 11. The force needed to pull the receiving interface element 18 together with the reservoir from the first to the second position is smaller than the retention force provided by the reservoir holding mechanism 6.

This manual step may be replaced by an automated mechanism, for example by a push-to-release mechanism comprising a spring which may be configured to move the receiving interface element 18 from the first position into the second position.

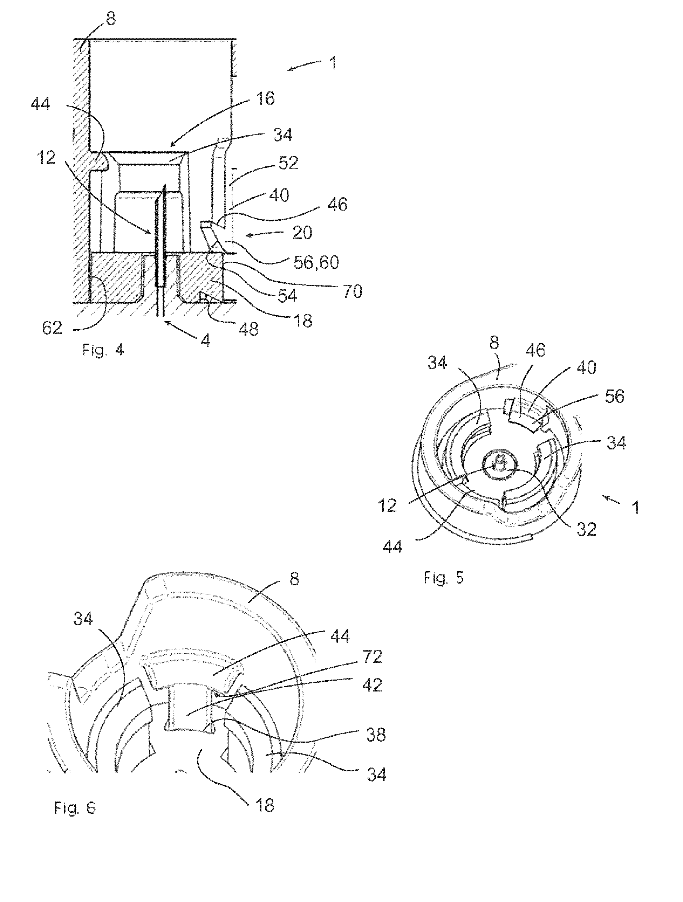

When the receiving interface element 18 is moved, a border area and/or edge 70 of the reservoir supporting interface 64 and the receiving interface element 18, respectively, is engaging a camming surface 54 of an elastic block element 40. The camming surface 54 and the elastic block element 40 are illustrated for example in figures 4, 7, 10 and 11. While the receiving interface element is moved from the first position towards the second position, the camming surface 54 is gliding on the edge 70 of the receiving interface element 18 and thus pushing the block element 40 sideways out of the movement path of the receiving interface element 18. As soon as the receiving interface element 18 reaches the second position, where the cannula 12 is disconnected from the reservoir 2 and the receiving interface element 18 is shielding the piercing tip 28, as illustrated in figures 7, 10 and 11, the camming surface 54 disengages with the receiving interface element 18 and the elastic block element 40 snaps towards the receiving interface element 18 so that a blocking portion 46 of the elastic block element 40 and a complementary blocking portion 48 of the receiving interface element 18 engage and lock the receiving interface element 18 in the second position.

In the embodiment shown in the figures, the blocking portion 46 and the complementary blocking portion 48 are oblique configured to ensure a safe and reliable blocking of the receiving interface element 18 and to avoid a random disengagement between the two portions 46, 48.

Even if the blocking portion 46 and the complementary blocking portion 48 are shown in an oblique shape other shapes such as a parallel or orthogonal in view of a direction of movement of the blocking portion 46 may be possible.

In the embodiments illustrated the elastic block element 40 further comprises an elastic cantilever arm 52, which is connected to the body portion 8, and a hook 56. The hook 56 comprising the camming surface 54 and the blocking portion 46 is arranged at a free end 60 of the elastic cantilever arm 52. The elastic arm 52 ensures an elastic snapping of the blocking portion 46 towards the complementary blocking portion 48 when the receiving interface element 18 reaches the second position.

In the embodiments illustrated, the elastic block element 40 comprises the elastic cantilever arm 52 to ensure that the blocking portion 46 engages or snaps towards the complementary blocking portion 48 as soon as the receiving interface element 18 reaches the second position. Other solutions are however possible.

The blocking mechanism 20 may for example comprise a bolt (not shown) comprising the blocking portion 46, said bolt being configured to be activated by a pre-stressed spring (not shown) as soon as the receiving interface element 18 reaches the second position. In such a configuration the camming surface 54 may for example be arranged at the end of the bolt directed towards the receiving interface element 18 so that the edge 70 of the receiving interface element 18 provides resistance to the spring force as long as the edge 70 is engaging the camming surface 54. When the receiving interface element 18 is in the second position, the camming surface 54 does no longer engage the edge 70 and the spring may move the bolt, which blocks the movement of the receiving interface element 18 from the second position back the first position.

In order to avoid a manual disengagement between the blocking portion 46 and the complementary blocking portion 48 by a patient or medical personnel, the blocking mechanism 20 may be shielded by a housing or the like (not shown), so that the blocking mechanism 20 is not accessible from the outside of the single-use drug reservoir 1.

When the receiving interface element 18 is moved from the first to the second position it is guided by a stopping and guiding mechanism 22 comprising a polarizing interface portion 42 formed on the body portion 8 and a complementary polarizing interface portion 38 formed on the receiving interface element 18, as illustrated in figures 2 and 3. Figures 2 and 3 are cross-sectional views cut along lines II-II and III-III, respectively, of figure 1 as seen in the direction of the arrows illustrated in figure 1. The complementary polarizing interface portion 38 and the polarizing interface portion 42 engage with one another during the whole process, thus in the first - and in the second position, ensuring that the rotary position of the receiving interface element 18 relative to the body portion 8 stays the same.

In figures 2 and 3 are further recesses 74 visible that extend through the receiving interface element 18, these recesses 74 being arranged next to the latching arms 34 on the inner side of the latter, thus towards the passage 68. The recesses 74 are also visible in figure 8.

In the embodiments illustrated, the complementary polarizing interface portion 38 is in the shape of a groove and the polarizing interface portion 42 is in the shape of a guide rail. The polarizing interface portion 42 extends from the support 14 of the drug transfer system 6 into the cavity 62 of body portion 8. The polarizing interface portion 42 may be shaped integrally with the body portion 8. The complementary polarizing interface portion 38 and the polarizing interface portion 42 may be arranged vice versa, thus the guide rail may be arranged on the receiving interface element 18 and the groove on the body portion 8.

At an end 72 of the polarizing interface portion 42 turned away from the support 14, a stop member 44 is arranged on the body portion 8. This stop member 44 ensures that the receiving interface element 18 cannot be move beyond the second position, so that the reservoir 2 may be disconnected from the docking system 6 by disengaging the latching arms 34 thus by overcoming the retention force, without destroying the reservoir 2. The stop member 44 is best illustrated in figures 3 and 6. When the receiving interface element 18 is moved beyond the second position, away from the cannula 12, the stop member 44 engages the reservoir supporting interface 64 and the receiving interface element 18, respectively, or at least a part of it, thus stopping the movement of the receiving interface element 18.

Since the docking system 6 and the stop member 44 allow the removal of the reservoir 2 without destruction of it, a reservoir 2 still comprising drug or drug component may be removed and re-used with another medical device or with a new single-use drug reservoir connector 1. Figure 10 shows such a reservoir 2 still comprising some drug or drug component, c.f. drug filling level indicated in the reservoir in figure 10. However, even a reservoir 2 that is completely empty with a plunger 50 pushed fully into a container 58 of the reservoir 2, as illustrated in figure 11 may be removed from the single-use drug reservoir connector 1.

The single-use drug reservoir connector 1 may be installed in a drug reconstitution device as described in CH 00337/12, incorporated herein by reference. In such a drug reconstitution device lyophilized drug (powder) is contained in a first reservoir and a liquid drug or solution is contained in second reservoir. It is possible that when the lyophilized powder is fully dissolved with a part of the liquid drug that there is still liquid drug in the second reservoir left. By using the single-use drug reservoir connector 1 between the reservoir or container and liquid transfer unit as shown in CH 00337/12, it is possible to remove the second reservoir and use it with a new liquid transfer unit to dissolve lyophilized powder contained in a new first reservoir.

The application of the single-use drug reservoir connector is however not limited to the above described example with the drug reconstitution device described in CH 00337/12. The present invention may be used in various applications and medical devices.

## Claims

1. A single-use drug reservoir connector including a drug transfer system (4) comprising a cannula (12), a docking system (6) and a blocking mechanism (20) comprising a blocking portion (46), the docking system being configured to receive and hold a reservoir (2), the docking system comprising a reservoir holding mechanism (16) and a receiving interface element (18) comprising a complementary blocking portion (48), wherein the receiving interface element is configured to be moved from a first position, in which the cannula (12) is fluidically connected to the reservoir, to a second position, in which the cannula is disconnected from the reservoir, and wherein the receiving interface element engages the blocking portion in the second position via the complementary blocking portion, the blocking portion being configured to prevent the receiving interface element from being moved from the second position back to the first position.

2. A single-use drug reservoir connector according to claim 1, **characterized in that** the complementary blocking portion (48) and the blocking portion (46) comprise oblique engaging surfaces oriented to strengthen engagement when pressure is applied to avoid inadvertent disengagement between the receiving interface element and the blocking portion when the receiving interface element is in the second position.

3. A single-use drug reservoir connector according claim 1 or 2, **characterized in that** the reservoir holding mechanism comprises at least one latching element (34) configured to releasably hold the reservoir.

4. A single-use drug reservoir connector according to the preceding claim, **characterized in that** the latching element (34) is integrally formed with the receiving interface element.

5. A single-use drug reservoir connector according to any of the preceding claims, **characterized in that** the cannula protrudes from the receiving interface element when it is in the first position and **in that** the cannula is shielded by the receiving interface element when it is in the second position.

6. A single-use drug reservoir connector according to any of the preceding claims, comprising a body portion (8) in which the receiving interface element is arranged.

7. A single-use drug reservoir connector according to the preceding claim, **characterized in that** the body portion comprises a polarizing interface portion (42) and the receiving interface element comprises a complementary polarizing interface portion (38) configured to interact with the polarizing interface portion during the movement of the receiving interface element from the first position to the second position.

8. A single-use drug reservoir connector according to any of the two direct preceding claims, **characterized in that** the body portion comprises a stop member (44) configured to prevent the receiving interface element from moving beyond the second position.

9. A single-use drug reservoir connector according to any of the preceding claims, **characterized in that** blocking mechanism includes an elastic block element (40) comprising the blocking portion.

10. A single-use drug reservoir connector according to the preceding claim, **characterized in that** the elastic block element comprises a camming surface (54), the camming surface being configured to interact with the receiving interface element.

11. A single-use drug reservoir connector according to any the two direct preceding claims, **characterized in that** the elastic block element comprises an elastic cantilever arm (52), the blocking portion and a camming surface (54) being arranged in the region of a free end (60) of the cantilever arm.

12. A single-use drug reservoir connector according to the preceding claim, **characterized in that** the free end (60) of the cantilever arm is shaped as a hook (56) comprising the camming surface and the blocking portion.
